# EUROPEAN PATENT SPECIFICATION

(11) **EP 2 327 368 B1**
(45) Date of publication and mention of the grant of the patent: **23.07.2014**
(21) Application number: 10189403.8
(22) Date of filing: 29.10.2010
(51) Int. Cl.: A61B 19/00, F16M 11/04, B60R 11/02, E05D 11/08, F16D 65/14

(54) **Locking apparatus and medical apparatus including the same**
Sperrvorrichtung und medizinische Vorrichtung damit
Appareil de blocage et appareil médical l'incluant

(30) Priority: 30.11.2009 KR 20090116863
(43) Date of publication of application: 01.06.2011
(73) Proprietor: MEDISON CO., LTD., Kangwon-do 250-875 (KR)
(72) Inventor: Ko, Jae Yong, Seoul (KR); Woo, Kyeong Gu, Gyeonggi-do (KR); Lee, Sun Ki, Seoul (KR)
(74) Representative: Schmid, Wolfgang

(56) References cited:
- GB-A- 799 577
- US-A- 4 736 916
- US-A- 6 021 607
- US-A1- 2005 282 673

## Description

### BACKGROUND OF THE INVENTION

### 1. Field of the Invention

The present invention relates to a locking apparatus and a medical apparatus including the same. More particularly, the present invention relates to a locking apparatus and a medical apparatus including the same that can selectively restrict rotation of devices, such as a control panel, display unit, and the like.

### 2. Description of the Related Art

Generally, an ultrasonic diagnostic apparatus refers to a non-invasive apparatus that irradiates an ultrasound signal from a surface of a patient body towards a target internal organ beneath the body surface and obtains an image of a monolayer or blood flow in soft tissue from information in the reflected ultrasound signal (ultrasound echo-signal). The ultrasonic diagnostic apparatus has been widely used for diagnosis of the heart, the abdomen, the urinary organs, and in obstetrics and gynecology due to various merits thereof such as small size, low price, real-time image display, and high stability through elimination of radiation exposure, as compared with other image diagnostic systems, such as X-ray diagnostic systems, computerized tomography scanners (CT scanners), magnetic resonance imagers (MRIs), nuclear medicine diagnostic apparatuses, and the like.

The ultrasonic diagnostic apparatus includes a cart-shaped main body for receiving main components of the diagnostic apparatus and a probe for transmitting and receiving ultrasound signals in association with the main body. Additionally, the diagnostic apparatus includes a control panel and a display unit mounted on the main body.

The control panel is located in front of the main body and provided with various switches and keys for inputting commands for manipulation of the apparatus. The display unit is mounted on top of the main body and displays an image of an ultrasonic diagnosis result.

The control panel and the display unit may be rotated in the right and left directions to improve user convenience. The control panel and the display unit are typically provided to the main body via swivel hinges.

The swivel hinge is disposed between the control panel or the display unit and the main body and rotatably couples the control panel or the display unit thereto, so that the control panel or the display unit can be rotated to adjust the direction thereof as desired, even when the main body is securely mounted at a certain location.

It should be noted that the above description is provided for understanding of the background of the invention and is not a description of a conventional technique well-known in the art.

The swivel hinge is required to generate a proper degree of friction, that is, a proper maximum static friction torque, upon rotation of a device, such as the control panel or the display unit. For example, if the maximum static friction torque of the swivel hinge is too high, an excessive force will be required to rotate the device, and if the maximum static friction torque of the swivel hinge is too low, the device is liable to rotate even by slight impact, so that a user cannot obtain satisfaction in manipulation of the device.

Since the maximum static friction torque of the swivel hinge is determined by several factors such as fastening force of the swivel hinge, elasticity of a spring, and the like, it is difficult to adjust the maximum static friction torque of the swivel hinge to a constant value. Moreover, since the preferred maximum static friction torques of the swivel hinges varies from user to user, the existing swivel hinges do not provide satisfaction in manipulation of the devices having the swivel hinges coupled thereto.

The US 2005/282673 A1 describes a pivot bearing unit, having a pneumatic braking device with a first and second bearing part, which are embodied as rotatable relative to one another, wherein the braking device is activatable by means of compressed air via a compressed air supply line. The braking device is provided with a brake hose, embedded in a bearing plate, in the first bearing part, and the brake hose expands as a result of subjection to compressed air with a motion in the direction of a free end and, via an axially movable switching ring, exerts a force on at least one brake block of the second bearing part, for transmitting a braking moment between the first and second bearing parts.

The US 4 736 916 A discloses a pan unit for a fluid tripod head, the pan unit includes a cylinder upon which a camera or other piece of equipment is to be mounted, and a piston slidably received within said cylinder. The piston is to be mounted on said tripod head, said piston and cylinder co-operating to provide a first working space within which a viscous liquid is to be located to damp relative movement between said piston and cylinder about the longitudinal axis thereof, the volume of said first working space being variable in order to adjust the viscous drag between said piston and cylinder.

Therefore, there is a need to solve such a problem.

### SUMMARY OF THE INVENTION

The present invention is conceived to solve the problem of the related art as described above, and an aspect of the invention is to provide a locking apparatus and a medical apparatus including the same that has improved structure to provide satisfaction in manipulation.

In accordance with one aspect of the invention, a locking apparatus with the features mentioned in claims 1 is provided. The fluid supply part includes a fluid supply passage communicating with the locking part; and wherein the syringe unit supplies the fluid to the locking part or receives the fluid discharged from the locking part through the fluid supply passage.

The locking part may be made of a rubber material.

The syringe unit may include a cylinder containing the fluid; and a piston unit reciprocating within the cylinder to supply the fluid to the locking part or to discharge the fluid from the locking part.

The syringe unit may further include a resilient member compressing the piston unit in a direction of supplying the fluid to lock the rotating part.

The resilient member may be provided to the cylinder and the piston unit.

In accordance with another aspect, a medical apparatus includes the locking apparatus and a medical instrument provided to the locking apparatus.

### BRIEF DESCRIPTION OF THE DRAWINGS

The above and other aspects, features and advantages of the invention will become apparent from the following description in conjunction with the accompanying drawings, in which:
- Fig. 1: is a perspective view of a medical apparatus in accordance with one embodiment of the present invention;
- Fig. 2: is a schematic diagram of a locking apparatus in accordance with one embodiment of the present invention;
- Fig. 3: shows operation of the locking apparatus shown in Fig. 2;
- Fig. 4: is a schematic diagram of a locking apparatus in accordance with another embodiment of the present invention; and
- Fig. 5: shows operation of the locking apparatus shown in Fig. 4.

### DETAILED DESCRIPTION OF THE EMBODIMENT

Embodiments of the invention will now be described in detail with reference to the accompanying drawings. It should be noted that the drawings are not to precise scale and may be exaggerated in thickness of lines or size of components for descriptive convenience and clarity only. Furthermore, terms used herein are defined by taking functions of the invention into account and can be changed according to the custom or intention of users or operators. Therefore, definition of the terms should be made according to overall disclosures set forth herein.

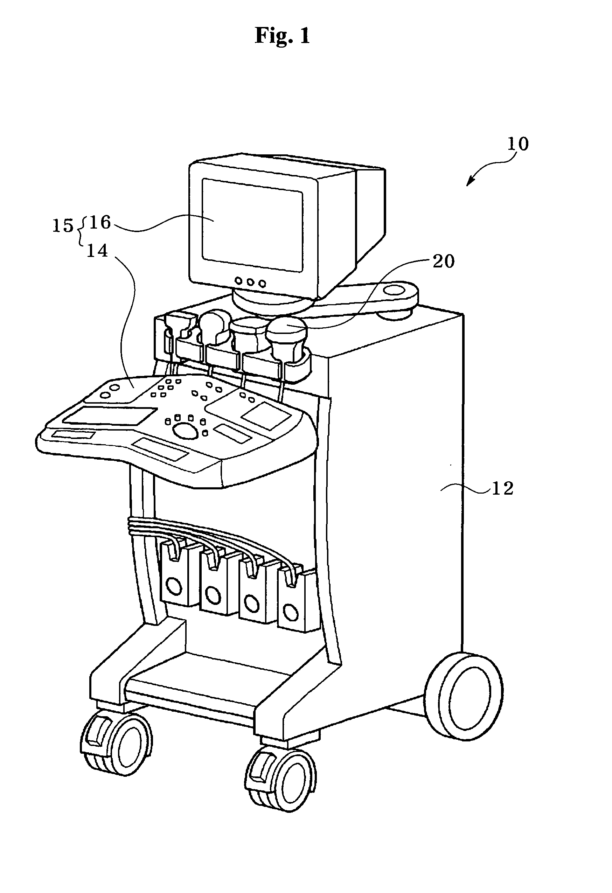

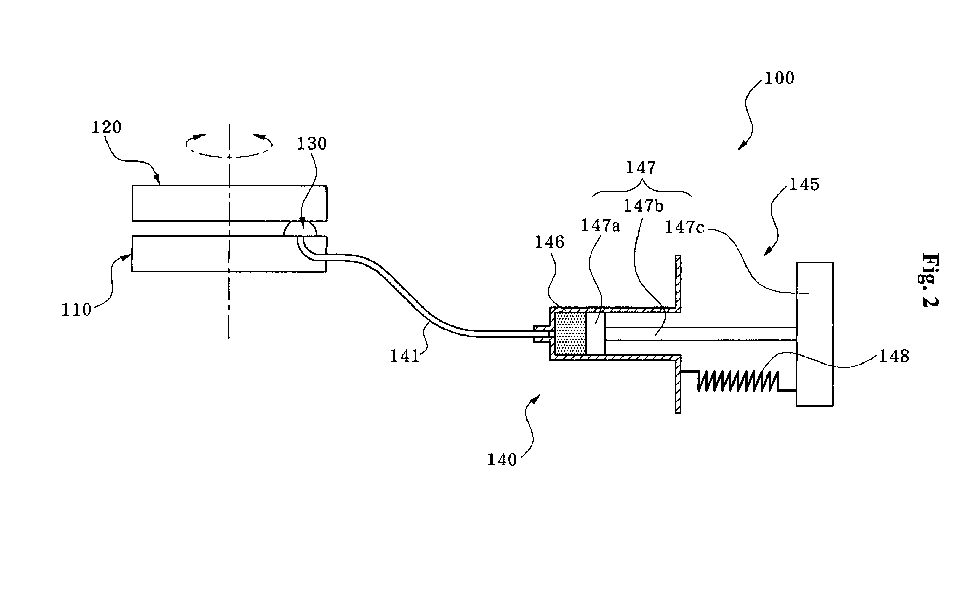

Fig. 1 is a perspective view of a medical apparatus in accordance with one embodiment of the invention, and Fig. 2 is a schematic diagram of a locking apparatus in accordance with one embodiment of the invention.

Referring to Fig. 1, a medical apparatus 10 of one embodiment includes a medical instrument 12, a control panel 14, and a display unit 16. In this embodiment, the medical apparatus 10 is an ultrasonic diagnostic apparatus that uses ultrasound waves for ultrasound diagnosis of a target, and the medical instrument 12 is illustrated as a main body of the medical apparatus 10.

The medical instrument 12 includes a beam former (not shown) for transmit-focusing ultrasonic signals transmitted through a probe 20 and receive-focusing ultrasonic signals received through the probe 20, a data creator (not shown) for creating frame data based on the signals output from the beam former, a processor (not shown) for generating a two-dimensional or three-dimensional internal image of a target based on the frame data, a storage (not shown) for storing data, and the like.

The control panel 14 includes a plurality of manipulation keys (not shown) and the like to drive the medical apparatus 10 or select a certain function thereof, and is electrically connected to the medical instrument 12. Preferably, the control panel 14 is located in front of the medical instrument 12 and is swivelly coupled to the medical instrument 12 via a locking apparatus 100 (see Fig. 2) to rotate in a right-left direction or in an up-down direction.

The display unit 16 serves to display ultrasound images generated by the medical instrument 12, operation states of the ultrasonic diagnostic apparatus 10, preset information for operating the ultrasonic diagnostic apparatus 10, and the like. Preferably, the display unit 16 is located on top of the medical instrument 12 and is coupled thereto via the locking apparatus 100 to rotate in the right-left direction or in the up-down direction.

Referring to Fig. 2, the locking apparatus 100 of one embodiment includes a stationary part 110, a rotating part 120, a locking part 130, and a fluid supply part 140.

The stationary part 110 is provided to the medical instrument 12 (see Fig. 1). The stationary part 110 is disposed between the medical instrument 12 and a device 15 (see Fig. 1) , such as the control panel 14 (see Fig. 1) , the display unit 16 (see Fig. 1), or the like, and secured to the medical instrument 12. For example, the stationary part 110 may have a disc shape.

The rotating part 120 is rotatably provided to the stationary part 110. For example, the rotating part 120 has a disc shape corresponding to the shape of the stationary part 110. The rotating part 120 is rotatably coupled at the center thereof to a rotational shaft (not shown) coupled to the center of the stationary part 110, and is separated a predetermined distance from the stationary part 110.

The device 15 is coupled to the rotating part 120. As such, the device 15 coupled the rotating part 120 may be provided to the medical instrument 12 to rotate in a rotating direction of the rotating part 120.

The locking part 130 is provided to the stationary part 110 to restrict movement of the rotating part 120. The locking part 130 is made of a material capable of expanding and contracting, and has a space therein to receive a fluid. In this embodiment, the locking part 130 is illustrated as being made of a rubber material and can be expanded or contracted by hydraulic pressure that is supplied into the locking part 130.

When the hydraulic pressure increases inside the locking part 130, the locking part 130 expands to compress the rotating part 120, movement of which is in turn restricted by friction between the rotating part 120 and the locking part 130, so that the rotation of the rotating device 120 and the device 15 coupled to the rotating device 120 is restricted.

The fluid supply part 140 supplies a fluid to the locking part 130 so as to allow the locking part 130 to restrict the movement of the rotating part 120. The fluid supply part 140 includes a fluid supply passage 141 and a syringe unit 145.

The fluid supply passage 141 communicates with the locking part 130. In this embodiment, the fluid supply passage 141 is illustrated as including a tube member that is connected at one end thereof to the locking part 130 and at the other end thereof to the syringe unit 145. The fluid supply passage 141 connects the locking part 130 to the syringe unit 145, thereby providing a path through which the fluid flows from the syringe unit 145 to the locking part 130 or vice versa.

The syringe unit 145 supplies the fluid to the locking part 130 or receives the fluid discharged therefrom through the fluid supply passage 141. The syringe unit 145 includes a cylinder 146, a piston unit 147, and a resilient member 148.

The cylinder 146 serves to contain the fluid. An interior of the cylinder 146 is formed with a space for receiving the fluid, in which the space communicates with the fluid supply passage 141.

The piston unit 147 is disposed inside the cylinder 146. The piston unit 147 reciprocates within the cylinder 146. The piston unit 147 serves to supply the fluid to the locking part 130 or discharge the fluid from the locking part 130 while reciprocating within the cylinder 146.

That is, when moving toward a side of the cylinder 146 to which the fluid supply passage 141 is connected, the piston unit 147 pushes the fluid out of the cylinder 146 into the fluid supply passage 141 so that the fluid received in the cylinder 146 can be supplied to the locking part 130. When moving in the opposite direction, the piston unit 147 forces the fluid supplied to the locking part 130 to be discharged therefrom.

In this embodiment, the piston unit 147 includes a piston 147a, a rod 147b, and a knob 147c. The piston 147a comes into close contact with an inner wall of the cylinder 146 to contact the fluid within the cylinder 146, and the knob 147c is located outside the cylinder 146 and connected to the piston 147a via the rod 147b.

According to this embodiment, reciprocation of the piston unit 147 for allowing the fluid to be supplied to the locking part 130 or discharged therefrom is accomplished by operation of the knob 147c.

In one example, the knob 147c may be manually operated by a user pulling the knob 147c. In another example, the knob 147c may be operated by power from an electric motor, such as a drive motor, a hydraulic or pneumatic cylinder, and the like, which is controlled by the medical instrument 12.

The resilient member 148 serves to compress the piston unit 147 in a direction, in which the fluid is supplied. The resilient member 148 forces the piston unit 147 to move in the direction of supplying the fluid, so that movement of the rotating part 120 is restricted by the expanded locking part 130. That is, the rotating part 120 enters a locked stated.

The resilient member 148 is provided to the cylinder 146 and the piston unit 147. In this embodiment, the resilient member 148 is illustrated as a coil spring that is coupled at one side thereof to the cylinder 146 and at the other side thereof to the knob 147c of the piston unit 147.

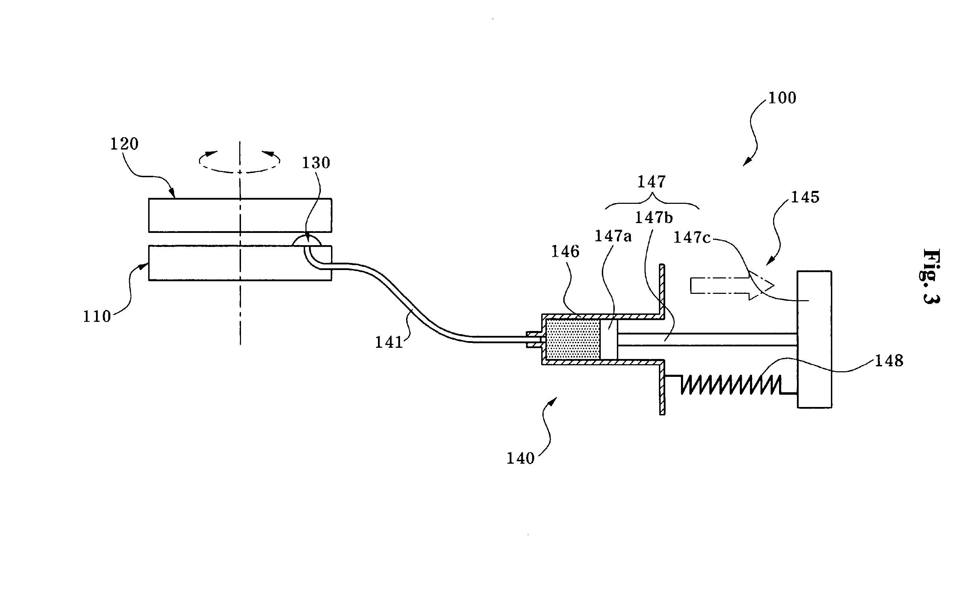

Fig. 3 shows operation of the locking apparatus shown in Fig. 2.

Operation and effect of the locking apparatus and a medical apparatus including the same according to the embodiment will now be described with reference to Figs. 2 and 3.

First, as shown in Fig. 2, with no external force applied to the syringe unit 145, that is, in a non-operative state of the knob 147c, the locking part 130 remains in an expanded state by hydraulic pressure of a fluid supplied from the fluid supply part 140, so that the rotating part 120 is locked by the locking part 130.

With the rotating part 120 locked by the locking part 130, the rotating part 120 and the device 15 coupled to the rotating part 120 are suppressed from rotating due to friction between the rotating part 120 and the locking part 130.

Then, as shown in Fig. 3, when the knob 147c is manipulated to move the piston unit 147 in an opposite direction to a direction in which the fluid is supplied, the fluid received in the locking part 130 is discharged to the fluid supply passage 141, so that the volume of the locking part 130 is reduced.

As such, when the volume of the locking part 130 is reduced to such a degree that the locking part 130 cannot compress the rotating part 120, the rotating part 120 is released from the locked state and can be freely rotated, and the device 15 coupled to the rotating part 120 can also be freely rotated together with the rotating part 120.

The locking apparatus 100 of the embodiment described above allows the locking part 130 to be expanded or contracted according to manipulation of a user so as to selectively restrict movement of the rotating part 120, so that the device 15 can be easily rotated without much effort or can be firmly secured according to the intention of the user, thereby providing satisfaction in manipulation of the device.

Further, the medical apparatus 10 including the locking apparatus 100 of the embodiment suppresses unintentional rotation of the device 15 by slight accidental impact, thereby preventing damage of the device 15, the medical instrument 12 and the like due to the unintentional rotation of the device and collision between the device 15 and the medical instrument 12 or other devices resulting therefrom.

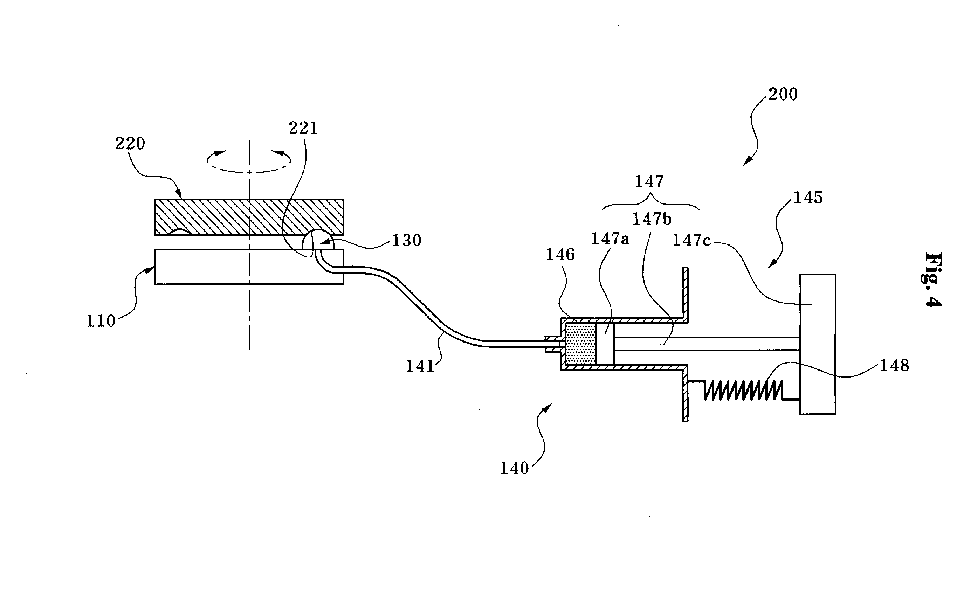

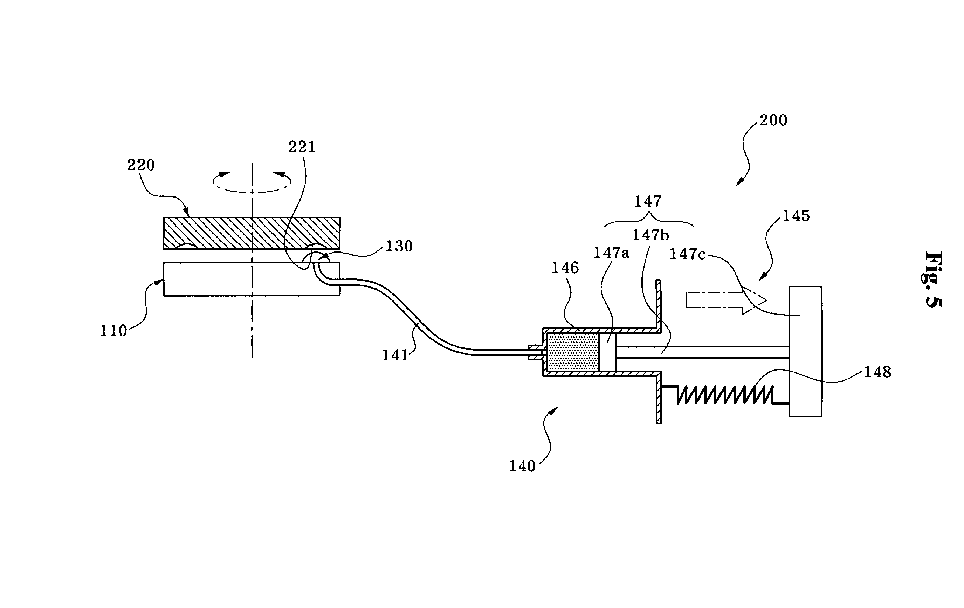

Fig. 4 is a schematic diagram of a locking apparatus according to another embodiment, and Fig. 5 shows operation of the locking apparatus of Fig. 4.

For descriptive convenience, the same or similar components of the embodiment as those of the above embodiment will be denoted by the same reference numerals, and a detailed description thereof will be omitted herein.

First, referring to Fig. 4, a locking apparatus 200 of this embodiment includes a rotating part 220 that has a fixing groove 221 formed on one side thereof with.

The fixing groove 221 is formed on the one side of the rotating part 220, that is, on a surface of the rotating part 220 which will be brought into contact with a locking part 130. In this embodiment, the rotating part 220 has a plurality of fixing grooves 221 on the one side thereof. The plural fixing grooves 221 are arranged along a track with which the locking part 130 will be brought into contact during rotation of the rotating part 220.

Here, the number of fixing grooves 221 and a distance between the respective fixing grooves 221 may be adjusted by taking into account the minimum rotating angle of the rotating part 220 that allows the direction of the device 15 (see Fig. 1) to be efficiently adjusted.

As shown in Fig. 4, the expanded locking part 130 is inserted into one of the fixing grooves 221. The locking part 130 inserted into the fixing groove 221 interferes with the rotating part 220 to thereby restrict movement of the rotating part 220. With the rotating part 220 locked by the locking part 130, the rotating part 220 and the device 15 coupled to the rotating part 220 enter a locked state and are suppressed from rotation.

Then, as shown in Fig. 5, when a knob 147c is manipulated to move a piston unit 147 in an opposite direction to the direction in which the fluid is supplied, the fluid received in the locking part 130 is discharged to a fluid supply passage 141, so that the volume of the locking part 130 is reduced.

As such, when the volume of the locking part 130 is reduced to allow the locking part 130 to be released from the fixing groove 221, the rotating part 220 is released from the locked state and can be freely rotated, and the device 15 coupled to the rotating part 220 can also be freely rotated together with the rotating part 220.

The locking apparatus 200 of this embodiment can further reliably secure the device 15 via coupling between the locking part 130 and the fixing groove 220 when securing the device 15.

As such, the locking apparatus according to the embodiments allows a locking part to be expanded or contracted according to manipulation of a user so as to selectively restrict movement of a rotating part, so that a device mounted on a medical apparatus via the locking apparatus can be easily rotated without much effort or can be firmly secured according to the intention of a user, thereby providing satisfaction in manipulation.

Further, the medical apparatus including the locking apparatus according to the embodiments suppresses unintentional rotation of a device thereon by slight accidental impact, thereby preventing damage of the device, a medical instrument, and the like due to the unintentional rotation of the device and collusion between the device and the medical instrument or other devices resulting therefrom.

Although some embodiments have been provided to illustrate the invention in conjunction with the drawings, it will be apparent to those skilled in the art that the embodiments are given by way of illustration only, and that various modifications and equivalent embodiments can be made without departing from the scope of the invention. The scope of the invention should be limited only by the accompanying claims.

## Claims

1. A locking apparatus (100; 200) comprising:
a stationary part (110);
a rotating part (120; 220) rotatably provided to the stationary part (110) ;
a locking part (130) provided to the stationary part (110) to restrict rotation of the rotating part (120; 220); and
a fluid supply part (140) providing a fluid to the locking part (130) to allow the locking part (130) to restrict the rotation of the rotating part (120; 220), **characterised in that** the fluid supply part (140) comprises a fluid supply passage (141) communicating with the locking part (130); and
a syringe unit (145) supplying the fluid to the locking part (130) or receiving the fluid discharged from the locking part (130) through the fluid supply passage (141).

2. The locking apparatus (100; 200) according to claim 1, **characterized in that** the locking part (130) is made of a rubber material.

3. The locking apparatus (100; 200) according to claim 2, **characterized in that** the syringe unit (145) comprises:
a cylinder (146) containing the fluid; and
a piston unit (147) reciprocating within the cylinder (146) to supply the fluid to the locking part (130) or to discharge the fluid from the locking part (130).

4. The locking apparatus (100; 200) according to claim 3, **characterized in that** the syringe unit (145) further comprises a resilient member (148) compressing the piston unit (147) in a direction of supplying the fluid to lock the rotating part (120; 220).

5. The locking apparatus (100; 200) according to claim 4, **characterized in that** the resilient member (148) is provided to the cylinder (146) and the piston unit (147).

6. A medical apparatus (10), **characterized by** comprising:
the locking apparatus (100; 200) according to any one of claims 1 to 5; and
a medical instrument (12) provided to the locking part (130) of the locking apparatus (100; 200).

## Patentansprüche

1. Sperrvorrichtung (100; 200), welche Folgendes aufweist:
ein stationäres Teil (110);
ein rotierendes Teil (120; 220), das drehbar an dem stationären Teil (110) angeordnet ist;
ein Verriegelungsteil (130), das an dem stationären Teil (110) angeordnet ist, um die Rotation des rotierenden Teils (120; 220) zu beschränken; und
ein Fluidzuführteil (140), das dem Verriegelungsteil (130) ein Fluid zuführt, um es dem Verriegelungsteil (130) zu erlauben, die Rotation des rotierenden Teils (120; 220) zu beschränken,
**dadurch gekennzeichnet, dass** das Fluidzuführteil (140) einen Fluidzuführkanal (141) aufweist, der mit dem Verriegelungsteil (130) in Verbindung ist; und
eine Spritzeneinheit (145), die dem Verriegelungsteil (130) das Fluid zuführt oder das von dem Verriegelungsteil (130) ausgegebene Fluid durch den Fluidzuführkanal (141) aufnimmt.

2. Sperrvorrichtung (100; 200) nach Anspruch 1,
**dadurch gekennzeichnet, dass** das Verriegelungsteil (130) aus einem Gummimaterial hergestellt ist.

3. Sperrvorrichtung (100; 200) nach Anspruch 2,
**dadurch gekennzeichnet, dass** die Spritzeneinheit (145) Folgendes aufweist:
einen Zylinder (146), der das Fluid enthält; und
eine Kolbeneinheit (147), die innerhalb des Zylinders (146) sich hin- und
herbewegt, um das Fluid zu dem Verriegelungsteil (130) zuzuführen oder das Fluid aus dem Verriegelungsteil (130) abzuleiten.

4. Sperrvorrichtung (100; 200) nach Anspruch 3,
**dadurch gekennzeichnet, dass** die Spritzeneinheit (145) des Weiteren ein elastisches Element (148) aufweist, welches die Kolbeneinheit (147) in einer Richtung der Zufuhr des Fluids komprimiert, um das rotierende Teil (120; 220) zu sperren.

5. Sperrvorrichtung (100; 200) nach Anspruch 4,
**dadurch gekennzeichnet, dass** das elastische Element (148) an dem Zylinder (146) und an der Kolbeneinheit (147) angeordnet ist.

6. Medizinische Vorrichtung (10),
**dadurch gekennzeichnet, dass** sie Folgendes aufweist:
die Sperrvorrichtung (100; 200) nach einem der Ansprüche 1 bis 5; und
ein medizinisches Instrument (12), das an dem Verriegelungsteil (130) der Sperrvorrichtung (100; 200) vorgesehen ist.

## Revendications

1. Dispositif de blocage (100 ; 200) comportant :
une partie stationnaire (110) ;
une partie rotative (120 ; 220) liée de façon rotative à la partie stationnaire (110) ;
une partie de verrouillage (130) liée à la partie stationnaire (110) pour limiter la rotation de la partie rotative (120 ; 220) ; et
une partie d'alimentation en fluide (140) agencée pour alimenter en fluide la partie de verrouillage (130) afin de permettre à la partie de verrouillage (130) de limiter la rotation de la partie rotative (120 ; 220),
**caractérisé en ce que** la partie d'alimentation en fluide (140) comprend un passage d'approvisionnement en fluide (141) pour délivrer un fluide qui communique avec la partie de verrouillage (130) ; et
une unité de seringue (145) qui alimente en fluide la partie de verrouillage (130) ou qui récupère le fluide évacué par la partie de verrouillage (130) à travers le passage d'approvisionnement en fluide (141).

2. Dispositif de blocage (100 ; 200) selon la revendication 1,
**caractérisé en ce que** la partie de verrouillage (130) est réalisée en un matériau à base de caoutchouc.

3. Dispositif de blocage (100 ; 200) selon la revendication 2,
**caractérisé en ce que** l'unité de seringue (145) comprend :
un cylindre (146) contenant le fluide ; et
une unité de piston (147) se déplaçant en va-et-vient à l'intérieur du cylindre (146) pour alimenter en fluide la partie de verrouillage (130) ou pour évacuer le fluide hors de la partie de verrouillage (130).

4. Dispositif de blocage (100 ; 200) selon la revendication 3, **caractérisé en ce que** l'unité de seringue (145) comprend en outre un organe élastique (148) qui comprime l'unité de piston (147) dans une direction d'alimentation en fluide pour bloquer la partie rotative (120 ; 220).

5. Dispositif de blocage (100; 200) selon la revendication 4, **caractérisé en ce que** l'organe élastique (148) est lié au cylindre (146) et à l'unité de piston (147).

6. Appareil médical (10) **caractérisé en ce qu'**il comporte le dispositif de blocage (100 ; 200) selon l'une quelconque des revendications 1 à 5 ; et un instrument médical (12) lié à la partie de verrouillage (130) du dispositif de blocage (100 ; 200).
